# EUROPEAN PATENT APPLICATION

(11) **EP 4 556 050 A1**
(43) Date of publication of application: **21.05.2025**
(21) Application number: 22951276.9
(22) Date of filing: 21.10.2022
(51) Int. Cl.: A61M 27/00, A61M 1/00

(54) **DRAINAGE TUBE GUIDE DEVICE**

(30) Priority: 12.07.2022 KR 20220085505
(71) Applicant: JSR Medical Co., Ltd., Daegu 42713 (KR); Kim, Jae Hwang, Daegu 42166 (KR)
(72) Inventor: KIM, Jae Hwang, Daegu 42166 (KR)
(74) Representative: Pfenning, Meinig & Partner mbB
(86) International application number: PCT/KR2022/016173
(87) International publication number: WO 2024/014621

(57) **Abstract**

A drainage tube guide device is provided, the device including a head part extending in one direction, a guide tube with a hollow formed therein and extending downward from the head part, and a drainage tube fixed to an end of the guide tube, the drainage tube being configured to dissect tissue at the front during introduction, and, once integrally coupled to the guide tube and reaching the target point, having its end removed.

## Description

### [Technical Field]

The present invention relates to a drainage tube guide device, and more particularly, to an integrated drainage tube guide device in which the drainage tube is integrally connected to the guide tube.

### [Background Art]

Drainage tubes are typically inserted into the human body at the surgical site to drain wound secretions, accumulated fluids, and post-surgical fluids, as well as to monitor fluid drainage and bleeding following surgery or procedures.

Typically, the drainage tube is fixed at the desired position by the operator. However, due to the movement (peristalsis) of the intestines, which occupy most of the abdominal cavity, the drainage tube often becomes dislodged from its fixed position within a few days after surgery. As a result, the drainage tube fails to fulfill its original purpose of draining unnecessary body fluids, blood, or secretions, effectively losing its function. The current method to address this issue involves suturing the drainage tube to the peritoneum within the abdominal cavity, but the results of this method have not shown significant improvement.

Recently, a method has been attempted to move and fix the drainage tube to the desired position via a route outside the abdominal cavity, that is, outside the peritoneum, which has shown improved effectiveness compared to the previous direct insertion of the drainage tube into the abdominal cavity.

The abdominal cavity is formed by the abdominal wall. The abdominal wall consists of the skin, subcutaneous fat layer, muscle layer enclosed by the anterior and posterior fascia, fascia itself, preperitoneal fat layer (or preperitoneal fat tissue plane), and peritoneum. Since the drainage tube is made of a soft material, an auxiliary tool is needed to move the drainage tube to the desired location through a route outside the peritoneum. Among the layers of the abdominal wall, the preperitoneal fat tissue plane is the least damaged and most easily accessible layer.

The preperitoneal fat tissue plane is a physically weak tissue layer, making it easy to separate. Therefore, among the possible extraperitoneal routes, passing through the preperitoneal fat tissue plane is the easiest and safest way to reach the target point.

However, the insertion of a drainage tube through the peritoneum outside the abdominal cavity is currently impossible with existing technology, and although the need for a guide or auxiliary device for this purpose has been raised, no existing drainage tubes or related devices satisfy this requirement.

### [Disclosure]

### [Technical Problem]

It is an object of the present invention to provide a drainage tube guide device capable of guiding a drainage tube to a target point by separating the extraperitoneal fat tissue plane, without causing damage to tissues or organs.

### [Technical Solution]

According to an embodiment, a drainage tube guide device is provided.

The drainage tube guide device includes a head part extended in one direction; a guide tube having a hollow formed therein and extending downward from the head part; and a drainage tube fixed to an end of the guide tube and configured to dissect tissue in front during introduction.

The head part may an inlet which communicates with the hollow of the guide tube so that a drainage tube is inserted into the guide tube from an upper surface or is removed from the guide tube.

The head part may include an identification portion protruding forward.

The head part may have one of a bar shape extending laterally, a spherical shape forming a curved surface, or a polyhedral shape forming a polygon.

The guide tube may include a straight portion extending in a straight direction from the head part; and an inclined portion extending upwardly at an angle to the straight portion.

The inclined portion may be curved at an angle equal to or greater than 25 degrees and less than or equal to 55 degrees relative to the straight portion.

The inclined portion may have a length greater than or equal to 1 cm and less than or equal to 5 cm.

The guide tube may have a length greater than or equal to 20 cm and less than or equal to 40 cm.

The drainage tube may include a hose portion inserted into the hollow of the guide tube, and a peeling portion coupled to an end of the hose portion and configured to be introduced while peeling tissue in the front and to be separated from the hose portion upon reaching a target area.

The peeling portion may include a fixing portion fixedly coupled to the end of the guide tube, and a protruding portion formed to protrude in the direction of insertion.

The fixing portion may include a fitting groove into which the end of the guide tube is inserted, and a fitting wing designed to fix the end of the guide tube.

The protruding portion may have a rounded shape at an end, which is not sharp.

### [Advantageous Effects]

It is advantageous for the drainage tube to be guided to the target point by separating the extraperitoneal fat tissue plane without damaging tissues or organs.

The insertion and procedure of the drainage tube through an extraperitoneal route allow for fluid drainage over the desired period, without the drainage tube dislodging due to intestinal movement or physical activity after surgery.

### [Description of Drawings]

FIG. 1 is a perspective view of a drainage tube guide device according to an embodiment;
FIGS. 2 and 3 are diagrams illustrating the head portion of the drainage tube guide device according to an embodiment;
FIGS. 4 and 5 are diagrams illustrating the structure of the drainage tube guide device, in which the drainage tube is connected to the guide tube 200 according to an embodiment; and
FIGS. 6 and 7 are diagrams illustrating the structure of the drainage tube guide device, in which the drainage tube is detached according to an embodiment.

### [Mode for Invention]

The embodiments of the present invention will be described in detail hereinafter with reference to the accompanying drawings to facilitate implementation by those skilled in the art within the relevant technical field. However, the present invention can be embodied in various forms, and is not limited to the embodiments described herein. For the purpose of clarity, components that are not directly related to the description have been omitted from the drawings, and like parts are designated by like reference numerals throughout the specification.

Throughout the specification, when it is stated that a part 'includes' a certain component, this means that, unless otherwise explicitly stated, other components may be included as well, rather than excluding other components.

The present invention can be modified in various ways and can have several embodiments, but specific embodiments are described in detail with reference to the drawings. This is not intended to limit the invention to a particular embodiment but should be understood to encompass all modifications, equivalents, or substitutions that fall within the spirit and scope of the present invention for connecting and/or fixing structures extending in different directions.

The terminology used in this specification is employed merely to describe specific embodiments and is not intended to limit the scope of the present invention. The singular forms are intended to include the plural forms as well unless the context clearly indicates otherwise.

In addition, detailed descriptions of well-known technologies may be omitted in the disclosure to avoid obscuring the subject matter of the disclosure.

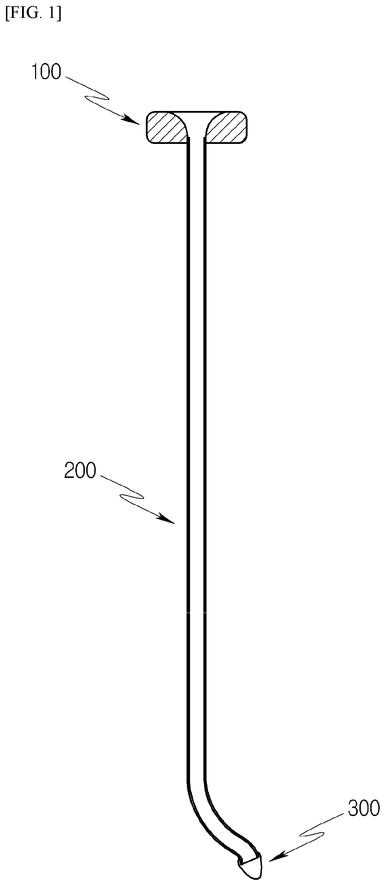

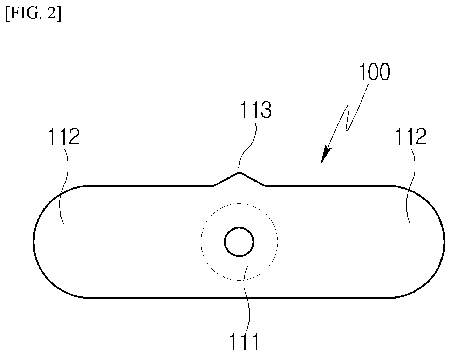

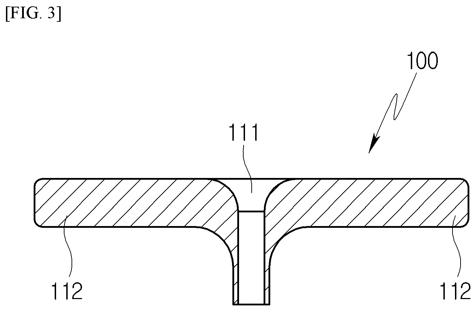

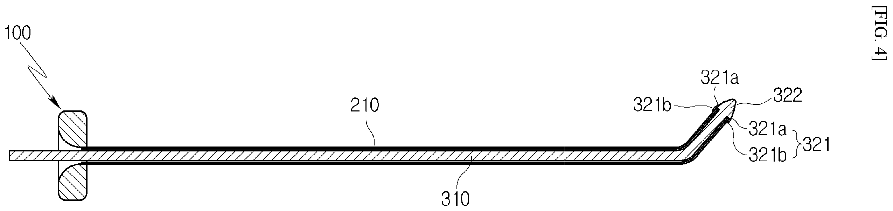

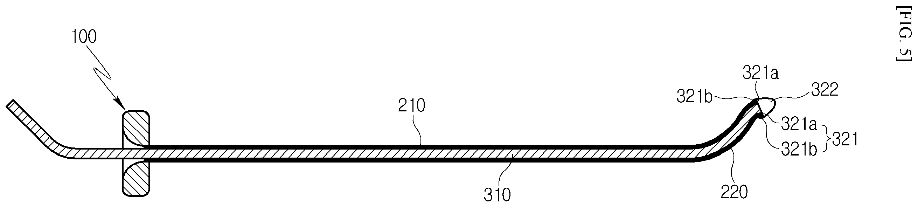

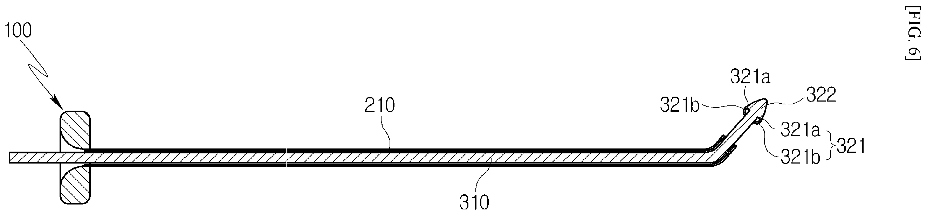

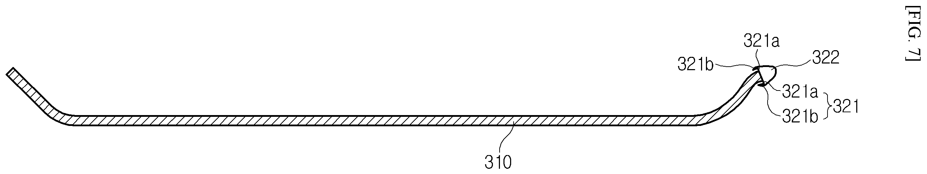

FIG. 1 is a perspective view of a drainage tube guide device according to an embodiment; FIGS. 2 and 3 are diagrams illustrating the head portion of the drainage tube guide device according to an embodiment; FIGS. 4 and 5 are diagrams illustrating the structure of the drainage tube guide device, in which the drainage tube is connected to the guide tube 200 according to an embodiment; and FIGS. 6 and 7 are diagrams illustrating the structure of the drainage tube guide device, in which the drainage tube is detached according to an embodiment.

With reference to FIGS. 1 to 4, the drainage tube guide device according to an embodiment includes a head part 100, a guide tube 200, and a drainage tube 300.

The head part 100 may be formed as a bar shape extending in both directions, according to an embodiment.

According to an embodiment, the head part 100 may include an inlet 111 which communicates with the hollow of the guide tube 200 so that the drainage tube 300 is inserted into the guide tube 200 from an upper surface of the head part 100 or the drainage tube 300 is removed from the guide tube 200.

According to an embodiment, the inlet 111 may be formed to communicate with the hollow of the guide tube 200.

According to an embodiment, the handle portion 112 of the head part 100 may be formed in various shapes as shown in the drawings, it may have a cylindrical shape or a spherical shape, which extends in both directions, or it may be any one of a polyhedron with more than six faces.

According to an embodiment, the handle portion 112 may be formed in the shape of a bar or rod extending in both directions to serve as a handle and may further include a plurality of grip grooves (not shown) that are inwardly recessed and spaced apart from each other for the user to grip.

The grip grooves (not shown) may be formed in a plurality of inwardly curved grooves, corresponding to the shape of fingers, to provide a comfortable grip when the user holds the handle portion 112.

According to an embodiment, the head part 100 may include an identification portion 113 that protrudes forward. The direction of protrusion of the identification portion 113 and the direction of protrusion of the protrusion portion 322 may coincide, thereby preventing the protrusion portion 322 from being inserted in the opposite direction during abdominal cavity insertion.

The abdominal cavity is formed by the abdominal wall. The abdominal wall is composed of the skin, subcutaneous fat layer, muscle layer enclosed by the anterior and posterior fascia, preperitoneal fat layer, and peritoneum.

According to an embodiment, the guide tube 200 may be inserted along the abdominal wall.

According to an embodiment, the guide tube 200 may be formed in a hollow shape communicating with the inlet 111 and may guide the drainage tube inserted through the inlet 111.

According to an embodiment, the guide tube 200 may extend downward from the bottom surface of the head part 100.

The longitudinal axis of the abdominal cavity typically measures about 30 to 50 cm, while the transverse axis is about 20 to 30 cm, with the radius of the abdominal cavity cross-section varying from 10 to 20 cm depending on body shape.

The longest axis of the abdominal cavity extends from the diaphragm above the liver to the end of the rectum (levator ani muscle) and is approximately 30 to 50 cm in length depending on an adult's body size.

Considering the width and depth of the abdominal cavity in all adults, the guide tube 200, according to an embodiment, may have a length of 20 to 40 cm.

According to an embodiment, the guide tube 200 may be made of a plastic material with a rigidity that does not allow bending, or of a metallic material.

According to an embodiment, the guide tube 200 may be made of a soft plastic material capable of bending.

According to an embodiment, the guide tube 200 may include a straight portion 210 extending in a straight line from the head part 100 and an inclined portion 220 extending upwardly at an angle from the straight portion 210.

According to an embodiment, the inclined portion 220 may be curved at an angle of 25 to 55 degrees relative to the straight portion 210.

According to an embodiment, the inclined portion 220 may have a length of 1 to 5 cm.

With reference to FIGS. 4 to 7, the drainage tube 300 may, according to an embodiment, be fixed at an end of the guide tube 200 and may be introduced while peeling tissue from the front. Once the target area is reached, the head part 100 and guide tube 200 may be separated from the drainage tube 300 by the user.

According to an embodiment, the drainage tube 300 may include a hose portion 310 and a peeling portion 320.

According to an embodiment, the hose portion 310 is initially inserted into the hollow of the guide tube 200 and, after entering the abdominal cavity and reaching the target area, may be separated from the guide tube 200.

According to an embodiment, the peeling portion 320 may be coupled to an end of the hose portion 310, inserted while peeling the tissue in front, and separated from the hose portion 310 upon reaching the target area. Upon reaching the target area, the peeling portion 320 may be removed by the user, and only the hose portion 310 may remain at the target area.

According to an embodiment, the peeling portion 320 may have an arrowhead shape.

According to an embodiment, the peeling portion 320 may include a fixing portion 321 and a protruding portion 322.

According to an embodiment, the fixing portion 321 may be fixedly coupled to the end of the guide tube 200.

According to an embodiment, the fixing portion 321 may include a fitting groove 321a into which the end of the guide tube 200 is inserted and a fitting wing 321b that fixes the end of the guide tube 200.

According to an embodiment, the protruding portion 322 may be formed to protrude in the direction of insertion.

According to an embodiment, the protruding portion 322 may be formed to incline downward toward the distal end. That is, the protruding portion 322 may have a rounded shape with a non-sharp tip.

According to an embodiment, the protruding portion 322 may be formed in a wedge shape with a tip that is not sharp.

The blunt protruding portion 322 facilitates dissection of the abdominal extraperitoneal fat layer, allowing for reaching the target area without damaging tissues and organs.

According to an embodiment, the end of the guide tube 200, which is coupled with the peeling portion 320, may have a straight shape as shown in FIG. 4.

According to an embodiment, the end of the guide tube 200, which is coupled with the peeling portion 320, may be curved in the opposite direction (the insertion direction) to the curved direction of the inclined portion 220, as shown in FIG. 5.

The end of the guide tube 200 in FIG. 5 may facilitate easier movement toward the target area compared to the end of the guide tube 200 in FIG. 4, and may minimize the dissection of the preperitoneal fat tissue plane.

Although curved instruments have the advantage of being suitable for movement along the contours of the abdominal cavity, when a direction change is required to reach the target area, they may excessively dissect the preperitoneal fat tissue plane, potentially causing unexpected bleeding. Additionally, users may find it more challenging to operate curved instruments compared to straight instruments due to a lack of familiarity with their curved shape.

In contrast, the guide tube 200 of the present invention, with its straight shape and the distal end being protruded and inclined upward, allows for minimal dissection of the preperitoneal fat tissue plane during movement toward the target area and prevents bleeding caused by excessive dissection through the blunt protruding portion 322.

The guide tube 200 of the present invention has a length of 20 to 40 cm, and the inclined portion 220 may have a length of 1 to 5 cm and be curved at an angle of 25 to 55 degrees or less. These numerical ranges are intended to ensure that the guide tube can reach the most distant target area in the abdominal drainage tube insertion area, with the exact range varying based on differences in body shape. The insertion of an extraperitoneal drainage tube minimizes the movement of the drainage tube caused by peristalsis, enabling long-term, effective drainage of body fluids within the abdominal cavity and improving therapeutic outcomes compared to conventional methods, such as the insertion and fixation of an intraperitoneal drainage tube through the abdominal cavity.

The present invention relates to a drainage tube guide device including a blunt protruding portion 322, which, by peeling the extraperitoneal fat layer without damaging tissues and organs and reaching the target area, allows for the insertion of a drainage tube through the retroperitoneum to drain body fluids from the abdominal cavity, thereby making the process of inserting and fixing the drainage tube through the existing surgical site easier and improving therapeutic effectiveness.

The present invention relates to a drainage tube guide device, in which a drainage tube with a blunt protruding portion 322 is fixed to the guide tube 200, allowing for dissection of the extraperitoneal fat layer without damaging tissues and organs, reaching the target area, and removing the guide tube 200 while holding the peeling portion 320 with another instrument, without the need to insert a separate drainage tube. The length of the drainage tube 300 can be adjusted by cutting off the excess drainage tube, including the peeling portion 320, with scissors.

After the drainage tube 300 is integrally coupled to the guide tube 200 and reaches the target area, the guide tube 200 can be removed, reducing the surgical time and improving the surgeon's efficiency. Since the drainage tube 300 reaches the target area while being integrally coupled to the guide tube 200, one step is eliminated compared to other devices, where the guide tube is inserted first, and the drainage tube is subsequently inserted into the guide tube.

Although the embodiments of the present invention have been described in detail above, the scope of the present invention is not limited thereto, and various modifications and improvements made by those skilled in the art using the basic concepts of the present invention defined in the following claims also fall within the scope of the present invention.

Those skilled in the art will appreciate that the present invention can be implemented in various forms without departing from the spirit and scope of the invention.

Therefore, the disclosed methods should be considered from a descriptive, rather than a restrictive, perspective. The scope of the present invention is not defined by the foregoing description but is defined in the patent claims, and all differences within the equivalent scope should be interpreted as included in the present invention.

## Claims

1. A drainage tube guide device comprising:
a head part extended in one direction;
a guide tube having a hollow formed therein and extending downward from the head part; and
a drainage tube fixed to an end of the guide tube and configured to dissect tissue in front during introduction.

2. The drainage tube guide device of claim 1, wherein the head part comprises an inlet which communicates with the hollow of the guide tube so that a drainage tube is inserted into the guide tube from an upper surface or is removed from the guide tube.

3. The drainage tube guide device of claim 1, wherein the head part comprises an identification portion protruding forward.

4. The drainage tube guide device of claim 1, wherein the head part has one of a bar shape extending laterally, a spherical shape forming a curved surface, or a polyhedral shape forming a polygon.

5. The drainage tube guide device of claim 1, wherein the guide tube comprises:
a straight portion extending in a straight direction from the head part; and
an inclined portion extending upwardly at an angle to the straight portion.

6. The drainage tube guide device of claim 5, wherein the inclined portion is curved at an angle equal to or greater than 25 degrees and less than or equal to 55 degrees relative to the straight portion.

7. The drainage tube guide device of claim 5, wherein the inclined portion has a length greater than or equal to 1 cm and less than or equal to 5 cm.

8. The drainage tube guide device of claim 1, wherein the guide tube has a length greater than or equal to 20 cm and less than or equal to 40 cm.

9. The drainage tube guide device of claim 1, wherein the drainage tube comprises:
a hose portion inserted into the hollow of the guide tube; and
a peeling portion coupled to an end of the hose portion and configured to be introduced while peeling tissue in the front and to be separated from the hose portion upon reaching a target area.

10. The drainage tube guide device of claim 9, wherein the peeling portion comprises:
a fixing portion fixedly coupled to the end of the guide tube; and
a protruding portion formed to protrude in the direction of insertion.

11. The drainage tube guide device of claim 10, wherein the fixing portion comprises:
a fitting groove into which the end of the guide tube is inserted; and
a fitting wing designed to fix the end of the guide tube.

12. The drainage tube guide device of claim 10, wherein the protruding portion has a rounded shape at an end, which is not sharp.
